# EUROPEAN PATENT SPECIFICATION

(11) **EP 1 698 290 B1**
(45) Date of publication and mention of the grant of the patent: **04.11.2009**
(21) Application number: 06450030.9
(22) Date of filing: 02.03.2006
(51) Int. Cl.: A61B 17/32, B24B 3/36, B24B 1/00, A61B 17/00

(54) **Method of manufacturing an Arthroscopic shaver with two pass inner blade**
Methode zur Herstellung eines Arthroskopischen Resektionsinstruments mit in zwei Schritten wirkender innerer Klinge
Méthode de fabrication d'un moyen de résection arthroscopique avec lame intérieure à deux pas

(30) Priority: 02.03.2005 US 657420 P
(43) Date of publication of application: 06.09.2006
(73) Proprietor: Arthrex, Inc., Naples, FL 34108-1945 (US)
(72) Inventor: Van Wyk, Robert Allen, Largo, Florida 33777 (US)
(74) Representative: Kopecky, Helmut

(56) References cited:
- EP-A- 0 276 478
- EP-A- 0 800 793
- US-A- 4 543 857
- US-A- 4 598 710
- US-A1- 2005 065 538
- US-B1- 6 217 598

## Description

### FIELD OF THE INVENTION

The present invention relates to arthroscopic surgery and, more particularly, to a method of manufacturing a shaver blade for arthroscopic surgery.

### BACKGROUND OF THE INVENTION

Resection of tissue by an arthroscopic shaver blade is accomplished by cooperative interaction between the edges of the inner and outer cutting windows. As the inner and outer windows come into alignment, tissue is sucked into the opening formed by these windows. Continued rotation of the inner member causes the inner cutting edges to approach the outer cutting edges. Tissue in the cutting window between the inner and outer edges is either trapped between the edges or ejected from the window. Tissue trapped between the edges is either cut by the edges as they approach each other, or torn by the cutting edges as they pass and rotate away from each other. The resected tissue is aspirated from the site through the inner lumen of the inner tube.

When a shaver is used with a constant rotation imparted to the inner tube, tissue in close proximity to the window is sucked into the window and either resected or ejected from the window as described previously. Tissue which is ejected from the window, or the remaining tissue adjacent to a resected portion, is swept in the direction of the rotation.

When the cutting window is opened again by the rotation of the inner member, the amount of tissue which will be sucked into the window is diminished from that of the previous opening because of the directional "set" of the tissue. That is, because the tissue is already preferentially oriented in the direction of the rotation of the approaching inner cutting edge, it is difficult for that inner cutting edge to have sufficient "bite" to retain the tissue in the cutting window for resection. Because of this, arthroscopic shavers are generally used in an "oscillate" mode when cutting tissue. In the "oscillate" mode, the inner member is rotated in one direction for a predetermined number of revolutions, whereupon its rotation is reversed for the same predetermined number of revolutions. The inner cutting edges approach the tissue from alternating directions, therefore greatly increasing the relative portion of tissue that is sucked into the window and is resected rather than ejected.

Decreasing the relative portion of tissue ejected from the window may also be accomplished by increasing the sharpness of the cutting edge, and by adding teeth to either the inner cutting edges, or to the outer cutting edges, or to both. Increasing the edge sharpness may be accomplished by decreasing the included angle of the cutting edge, that is, by decreasing the angle formed by the machined surface of an inner or outer cutting edge and either respectively the inner surface of the outer tube, or the outer surface of the inner tube when viewed in a section view parallel to the tube axis. Sharpness may also be increased by decreasing the edge radius, and by decreasing the roughness of the surfaces over which tissue must slide during resection.

From EP 0 800 793 A1, a process for simultaneously shaping and sharpening a rotatable surgical shaver blade, especially a cutting window at the distal end of a tubular member of a rotary blade assembly, is known. The assembly is provided with a stationary elongated outer tube, having a cutting window at its distal tip and includes a rotatable elongated inner tube having a cutting means at its distal tip. The window in the outer tube has a curvilinear profile defined by a peripheral rim surrounded entirely by a land surface which is inclined relative to the rim. The inclination of the land produces an area which tapers from a full thickness, where the land is adjacent to the cylindrical wall of the outer tube, to a sharp edge around the periphery of the window. The curvilinear window is produced by a manufacturing process which creates the window opening with a sharpened periphery at the same time that the land surrounding the opening is inclined. The method for manufacturing utilizes electrochemical grinding of the distal ends of the tubular memberby a tool having a rotatable wheel with a perimeter tn the shape of a groove of predetermined arcuate profile.

Shavers having inner cutting edges with teeth are well known in the art. For example, U.S. Patent No. 5,217,479 to Shuler and U.S. Patent No. 5,269,798 to Winkler describe shavers having inner cutting edges with teeth, the teeth being formed by a two-axis "through-cutting" process such as wire electrical discharge machining (wire EDM), or by grinding with a wheel having a shaped periphery. The teeth help retain tissue within the window, so that the tissue can be cut by the low included angle of the outer cutting edges, as the inner and outer edges converge. The inner cutting edges do little cutting since the edge portions between the teeth form a very large included angle cutting edge. One shaver having inner cutting edges with teeth is the Gator™ shaver sold by Linvatec Corporation (Largo, Florida).

Other shaver blades have teeth on both the inner and outer cutting edges formed by a two-axis through-cutting process. The Cuda™ sold by Linvatec Corporation (Largo, Florida) and the Tomcat™ sold by Stryker Corporation (Kalamazoo, Michigan) have teeth on both the inner and outer cutting edges, the edges being formed by grinding using a wheel with a shaped periphery, or by wire EDM. The regions of the edges formed between the teeth have large included angles which are inefficient for cutting tissue. Shavers having these two-dimensionally shaped teeth on the inner and outer cutting edges separate tissue primarily by tearing, as the edges pass each other after closure of the cutting window. Such tearing is undesirable since the torn tissue may frequently become wrapped into the gap between the inner and outer tubes and cause clogging.

U.S. Patent No. 6,053,928 to Van Wyk et al. describes a shaver having a plurality of teeth on the laterally opposed cutting edges of an outer window, the cutting edges being symmetrical when viewed in a plane perpendicular to the axis of the tube. The cutting edges are formed so that, when viewed in any such plane, the edges have low included angles in the troughs between the teeth as well as on the teeth themselves. Because of their low included angle edges, these teeth penetrate tissue more easily than the previously described two-dimensional teeth and, therefore, they prevent more effectively ejection of tissue from the cutting window. For example, the Great White™ shaver sold by Linvatec Corporation, constructed in accordance with the principles of U.S. Patent No. 6,053,928 to Van Wyk, et al., is very efficient at removing tissue and experiences reduced clogging due to the sharpness the outer cutting edges.

While advanced tooth geometries such as those described in Patent No. 6,053,928 to Van Wyk et al. have been produced for outer cutting edges with teeth, corresponding improvements have not been made in the geometry of the inner cutting edges with teeth. Teeth on inner cutting edges generally have a simple, two-dimensional shape. That is, the cross-section of the tooth has a constant cross-section when viewed in a side elevational view. One exception is the Gator™ shaver sold by Linvatec Corporation. The inner cutting edge teeth of the Gator™ shaver are formed by a two-step process. The profile of the teeth is formed in the conventional through-cut manner. The teeth are then "sharpened" by removing material by Electrical Discharge Machining (EDM), to form a beveled surface on the portion of a tooth in contact with the tube inner lumen. The resulting pyramidal teeth are effective for penetrating tissue and preventing its ejection from the cutting window as the inner and outer cutting edges approach each other. However, this two-step approach to making teeth has drawbacks. EDM is used to remove the material to form the beveled surface because conventional machining processes are unable to produce the required geometry under production conditions. In turn, the EDM process has high consumable tooling costs as the electrode is eroded during use. Also, the surface produced by the EDM process is rough and inhibits easy penetration of a tooth into tissue.

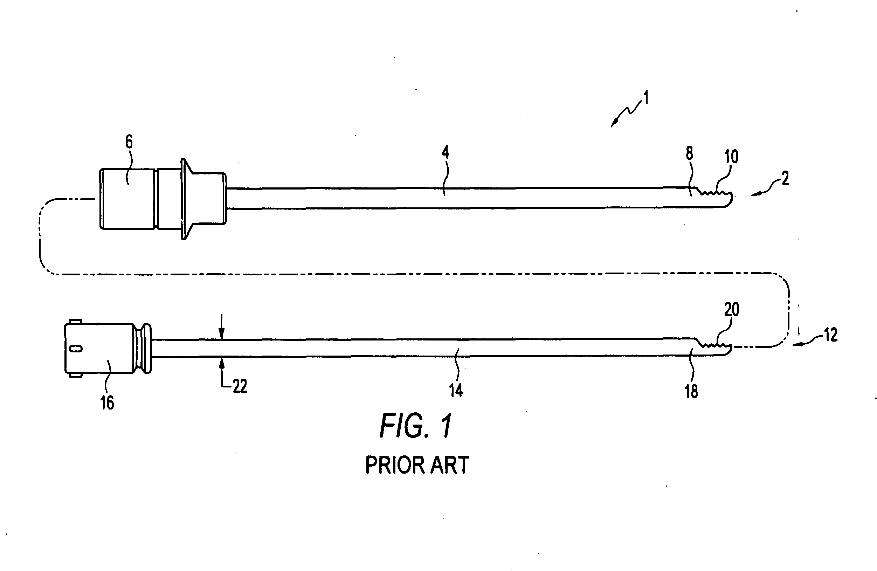

Figure 1 illustrates a prior art arthroscopic shaver 1 which has an outer assembly 2 having a metallic, elongated, tubular distal portion 4 and a proximal portion 6 forming a hub suitable for mounting in a shaver handpiece. Distal portion 4 has a distal end 8 forming cutting window 10. Shaver 1 also has an inner assembly 12 having a metallic, elongated, tubular distal portion 14 and a proximal portion 16 forming a hub suitable for transmitting rotational motion provided by a motor drive to inner assembly 12. Distal portion 14 has a distal end 18forming cutting window 20. Diameter 22 of the distal portion 14 of the inner assembly 12 is slightly less than the diameter of the inner lumen of the distal portion 4 of the outer assembly 2, so that the inner assembly 12 may be rotatably positioned therein for use.

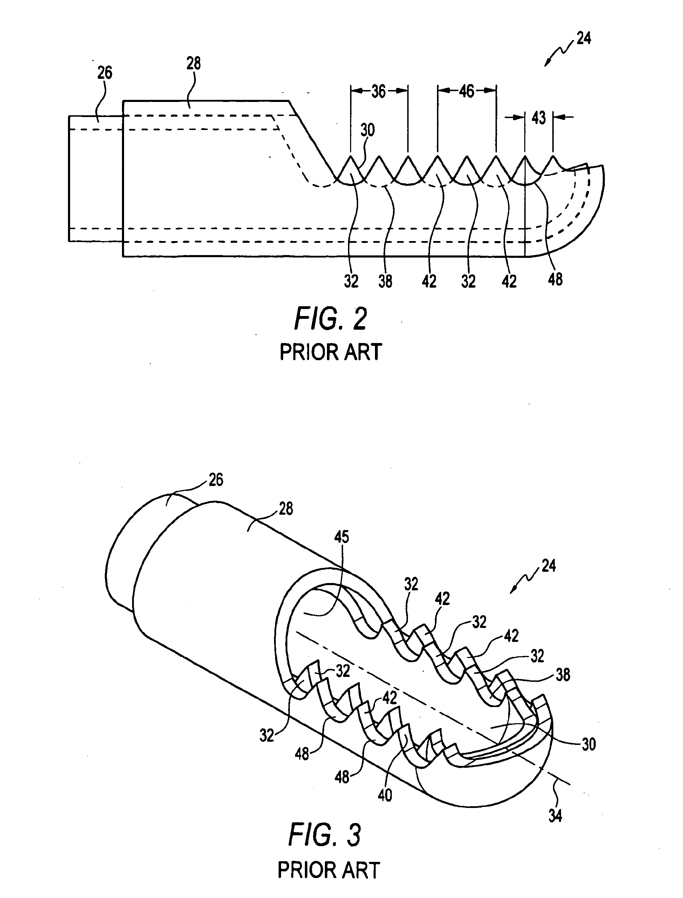

Figures 2 and 3 depict the distal end cutting windows of a prior art shaver 24, having inner tube 26 rotatably positioned within outer tube 28. Inner window 30 has a plurality of teeth 32 which are symmetrically placed about axis 34 when viewed in plan view. Teeth 32 are spaced apart by distance 36 and are separated by valleys 38. Outer window 40 has a plurality of teeth 42, which are symmetrically placed about axis 34 when viewed in a plan view. Teeth 42 are spaced apart by distance 46 which is approximately equal to distance 36, and are separated by valleys 48. Inner window teeth 32 are displaced axially from outer window teeth 42 by distance 43 equal to approximately half of distances 36 and 46, so that when the inner window is rotated toward the outer window, teeth 32 line up with valleys 48 of outer window 40, and outer window teeth 42 line up with valleys 38 of inner cutting window 30.

Referring to Figures 4 through 7, inner window 30 of inner tube 26 has teeth 32 formed by a two-dimensional, through-cutting process such as grinding or wire EDM. Teeth 32 have a constant cross-section as shown in Figure 5. Teeth 32 have knife-like cutting edges 47 having an included angle 49, the edges 47 of teeth 32 on opposite sides of cutting window 30 appearing collinear when viewed from the tube distal end (Figure 7).

During use, inner tube 26 is rotated within outer tube 28 generally in an oscillatory manner, as described previously. Suction supplied to lumen 45 pulls tissue into contact with, and partially into, the opening formed by angular alignment of windows 30 and 40. As teeth 32 of inner cutting window 30 engage the tissue, some teeth may penetrate the tissue and drag a portion of the tissue toward teeth 42 of outer cutting window 40. Some teeth 42 may also penetrate the tissue, thereby ensuring that a portion of the tissue will be trapped between the closing window edges and resected. Portions of the tissue which are not penetrated by the teeth will likely be ejected from the closing window by the approaching cutting edges and will not be resected. The efficiency or aggressiveness of a shaver is strongly affected by the ability of the inner cutting window edges to prevent tissue from being ejected from the closing aperture. This is strongly affected by the effectiveness of teeth 32 in penetrating a portion of the tissue which it encounters. When the rotation of the inner member is reversed, the process is repeated, this time on the opposite side of the cutting windows. As in the previous rotation, the efficiency of the cutting action is strongly affected by the ability of teeth 32 on inner window 30 to penetrate tissue in contact with, or partially drawn into, the cutting window.

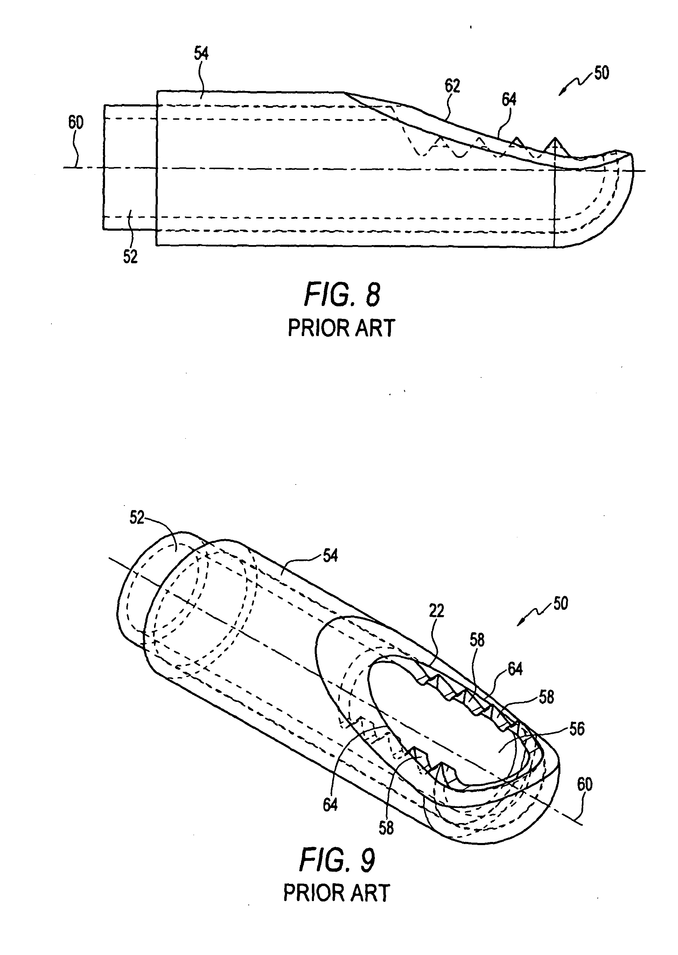

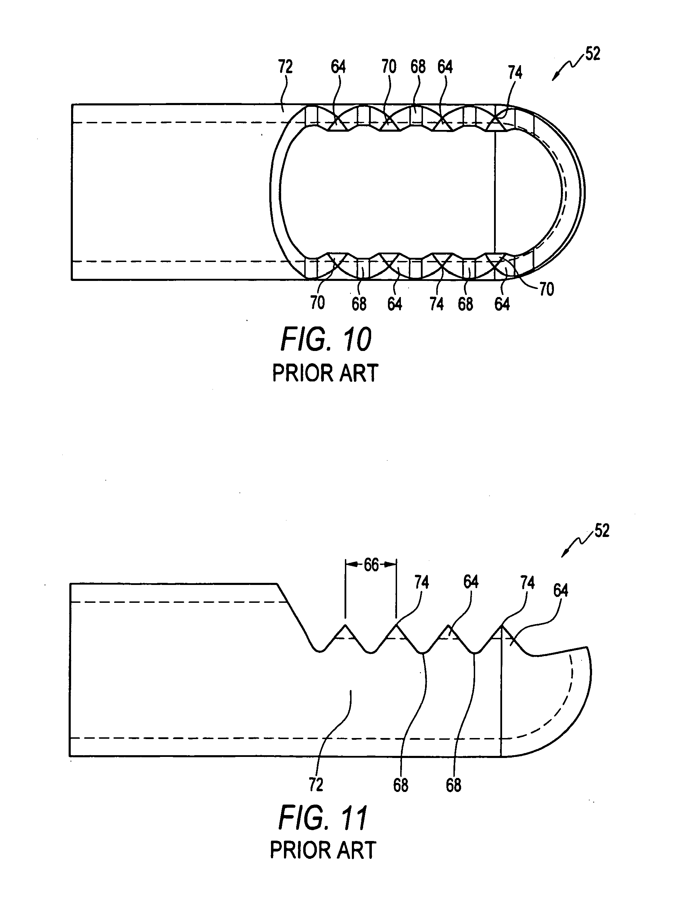

Figures 8 and 9 depict the distal end cutting windows of a Linvatec Corporation Gator™ prior art shaver 50, with inner tube 52 rotatably positioned within outer tube 54. Inner window 56 has a plurality of teeth 58 which are symmetrically placed about axis 60 when viewed in a plan view. Outer window 62 has low included angle cutting edges 64. As seen in Figures 10 through 13, teeth 64 are spaced apart by distance 66 on the distal portion of inner tube 52 of shaver 50, and are separated by valleys 68. Teeth 64 are formed in a two-step operation by cutting of the edge profile (Figure 11), followed by "sharpening" of the teeth through beveling of a portion 70 of each tooth, to produce teeth with pyramidal points 74 positioned at tube outer surface 72. As with the previous prior art device, teeth 64 penetrate tissue to aid in retention of the tissue between the inner and outer cutting edges as they approach. Beveling of teeth 64 increases the ease with which the teeth penetrate the tissue.

The beveling of the teeth, however, must be accomplished by Electrical Discharge Machining (EDM). Conventional machining processes such as milling or grinding are not able to access the portions of the teeth to be removed. EDM, however, uses a shaped electrode to remove tissue by vaporization. The electrode is eroded during the process and must be frequently replaced. This makes the consumable tooling costs high since the erosion is particularly pronounced in regions in which the electrode removes sharp corners. Also, because EDM removes material by melting and vaporization, it is difficult to produce a sharp point. The surfaces produced by EDM are also generally rough and present a high resistance to tissue sliding over the surface. This inhibits penetration of teeth 64 into tissue. Because the forming of teeth 64 is conducted in two processes on two different machine tools, a means must be provided for angular alignment of tube 52 during processing to ensure accurate placement of the bevel features on the teeth. Such alignment means frequently requires machining of features on the proximal end of the tube, an added cost when manufacturing the inner tube.

### SUMMARY OF THE INVENTION

Accordingly, it is an object of the present invention to produce an arthroscopic shaver blade with high resection efficiency due to advanced inner cutting edge tooth geometry.

It is also an object of the present invention to produce an arthroscopic shaver blade with high resection efficiency which has smooth finishes on all machined surfaces.

It is further an object of the present invention to produce a method for making the inner cutting edges for shaver blades with high resection efficiency due to advanced inner cutting edge tooth geometry.

It is further an object of the present invention to produce a method for making the inner cutting edges for shaver blades with high resection efficiency due to advanced inner cutting edge tooth geometry, which allows the edges to be produced complete on a single machine tool.

These and other objects are accomplished by the present invention which provides a method of manufacturing a shaver having increased efficiency because the inner cutting window has a plurality of teeth positioned along the lateral cutting edges, the teeth being configured for easy penetration into tissue to prevent ejection of tissue from the cutting window during closure. The inner cutting edges are formed completely by a predetermined sequence of positioning moves and grinding passes or operations on a multi-axis grinding machine. In one embodiment, the method results in teeth which are symmetrically disposed about the longitudinal tube axis when viewed in a plan view in a section normal to the longitudinal axis. In another embodiment, the teeth are asymmetrically disposed, viewed in a similar section.

These and other features and advantages of the invention will be more apparent from the following detailed description that is provided in connection with the accompanying drawings and illustrated exemplary embodiments of the invention.

### BRIEF DESCRIPTION OF THE DRAWINGS

Figure 1 is a disassembled view of a prior art arthroscopic shaver blade.

Figure 2 is a side elevational view of a distal portion of a prior art shaver blade.

Figure 3 is a perspective view of the shaver blade of Figure 2.

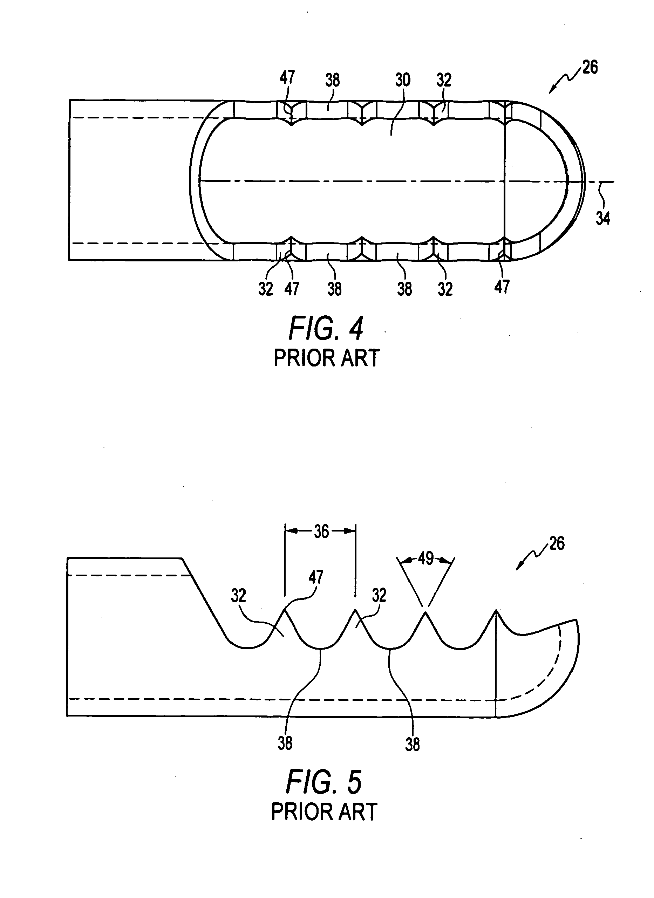

Figure 4 is a plan view of the distal end of the inner tube and cutting window of the shaver blade of Figure 2.

Figure 5 is a side elevational view of the shaver blade of Figure 4.

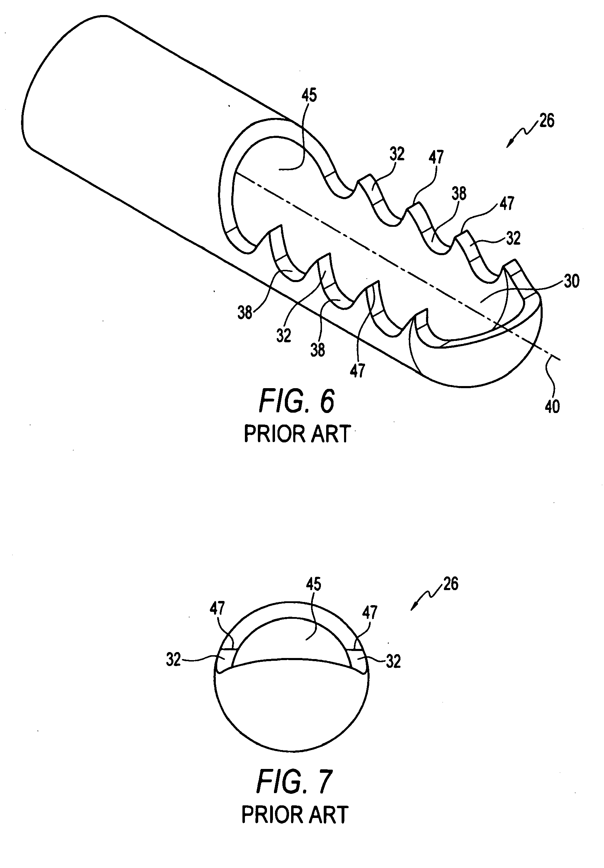

Figure 6 is a perspective view of the shaver blade of Figure 4.

Figure 7 is a distal end view of the shaver blade of Figure 4.

Figure 8 is a side elevational view of the distal end of another prior art shaver blade.

Figure 9 is a perspective view of the shaver blade of Figure 8.

Figure 10 is a plan view of the distal end of the inner tube and cutting window of the shaver blade of Figure 8.

Figure 11 is a side elevational view of the shaver blade of Figure 10.

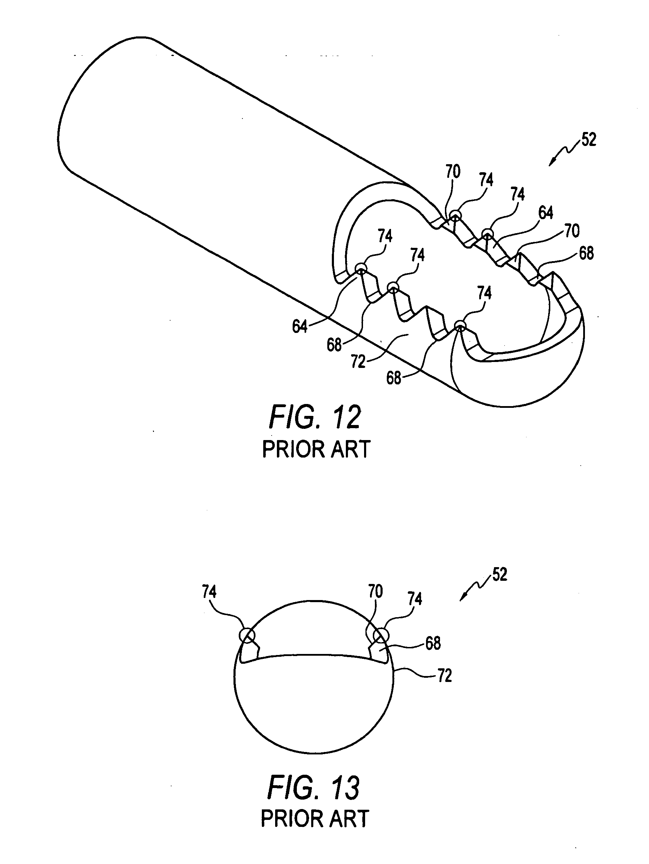

Figure 12 is a perspective view of the shaver blade of Figure 10.

Figure 13 is a distal end view of the shaver blade of Figure 10.

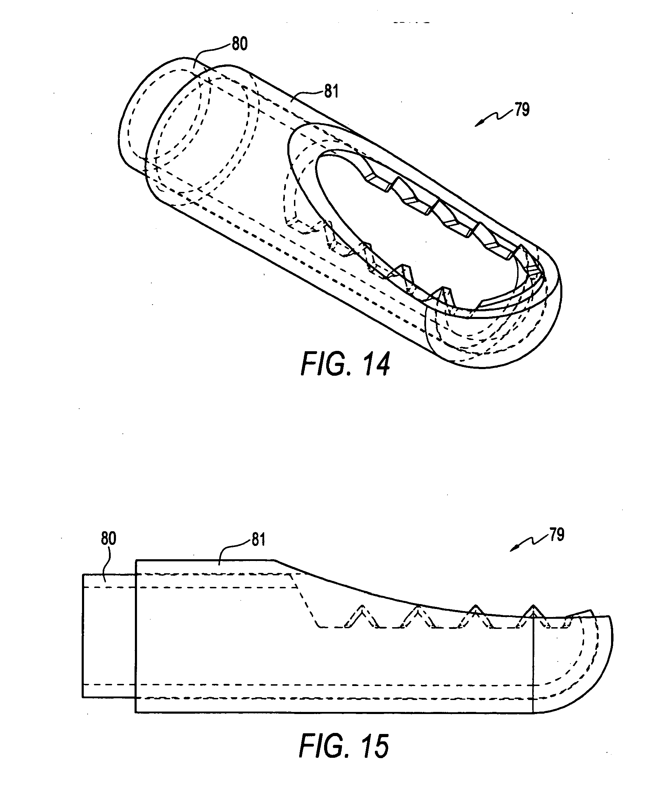

Figure 14 is a perspective view of a shaver blade having an inner cutting window formed in accordance with the present invention.

Figure 15 is a side elevational view of the shaver blade of Figure 14.

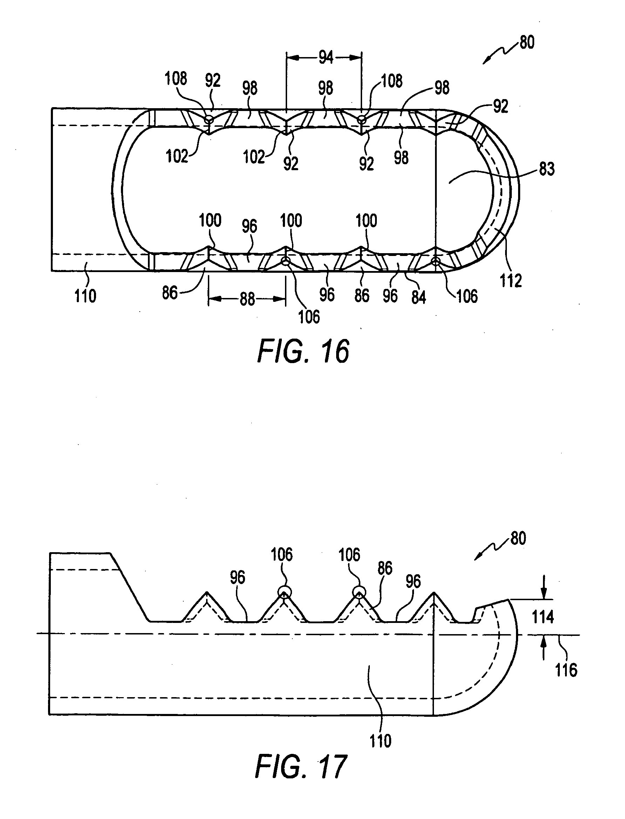

Figure 16 is a plan view of the distal end of a shaver inner tube distal end formed in accordance with the present invention.

Figure 17 is a side elevational view of the shaver of Figure 16.

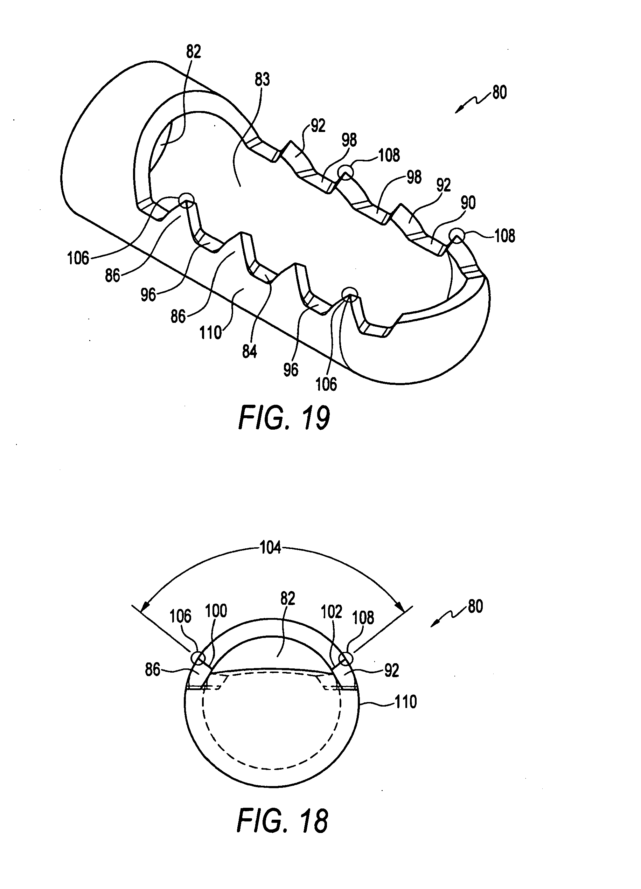

Figure 18 is a distal end view of the shaver of Figure 16.

Figure 19 is a perspective view of the shaver of Figure 16.

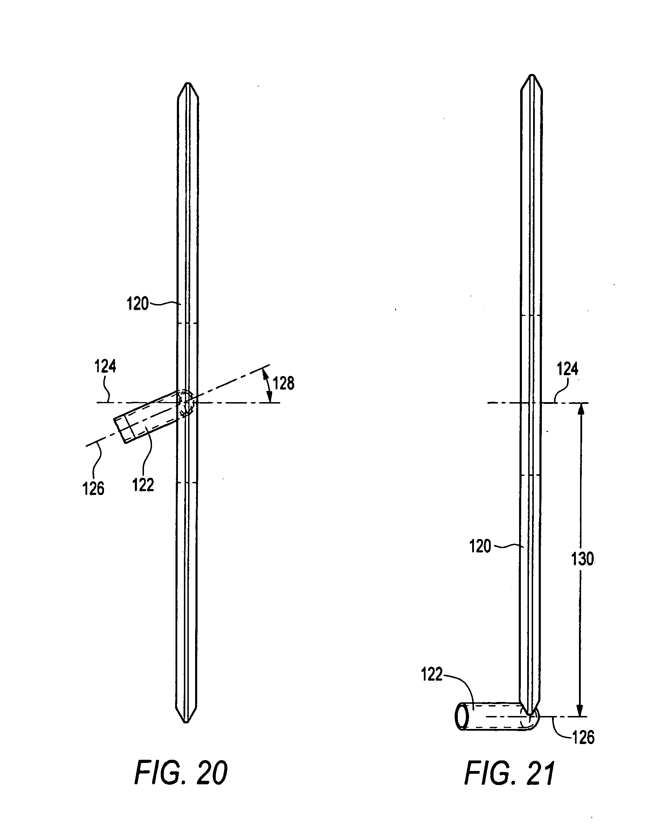

Figure 20 is a schematic plan view of a grinding wheel and tube showing their relationship when forming the shaver of Figure 16.

Figure 21 is a front elevational view of the structures of Figure 20.

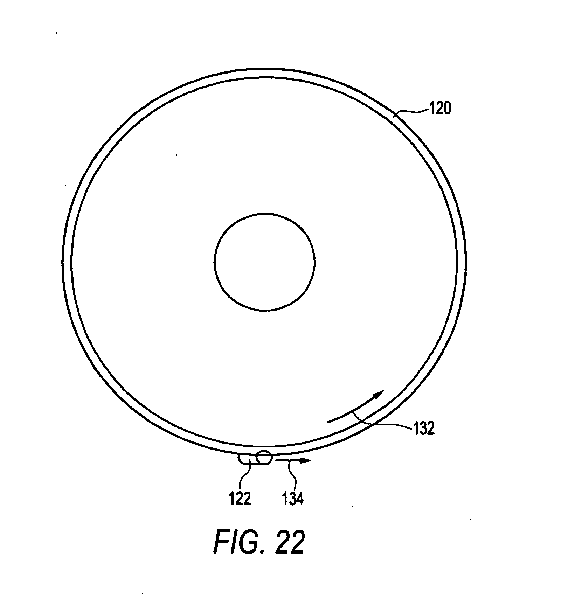

Figure 22 is an end view of the structures of Figure 20 in the direction of the axis of the grinding wheel of Figure 20.

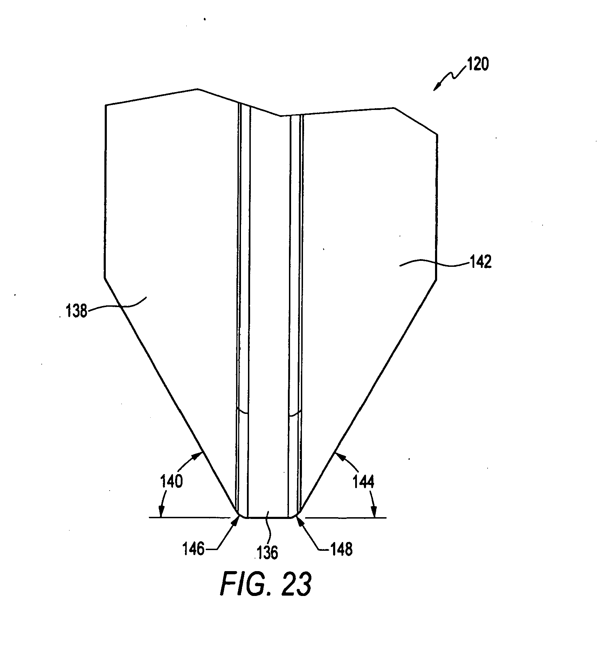

Figure 23 is an expanded tangential view of the periphery of the grinding wheel of Figure 20.

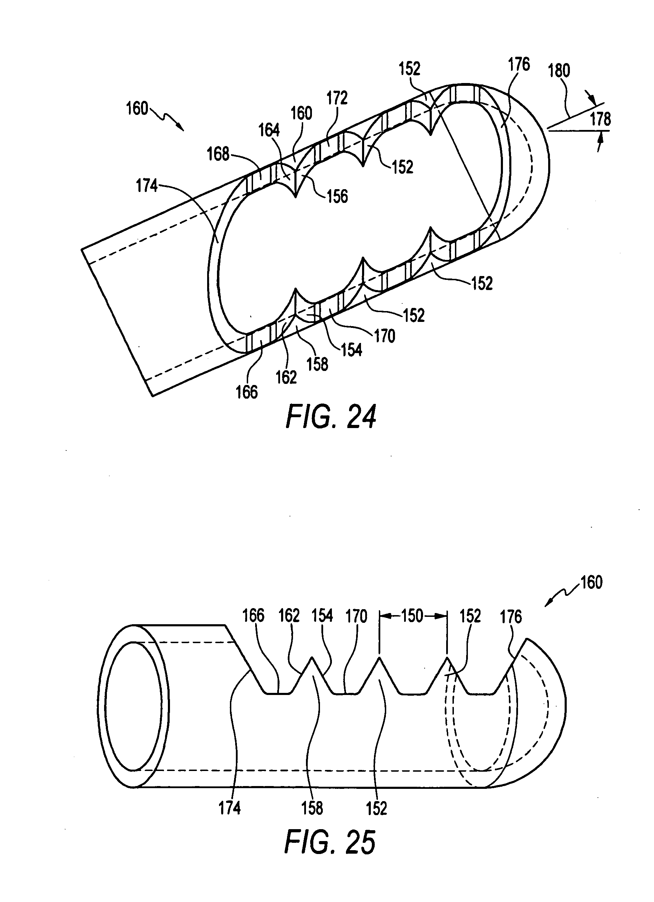

Figure 24 is a rotated plan view of the distal portion of a partially formed shaver inner cutting window formed in accordance with the present invention.

Figure 25 is a front elevational view of the rotated shaver of Figure 24 showing a partially formed tooth profile.

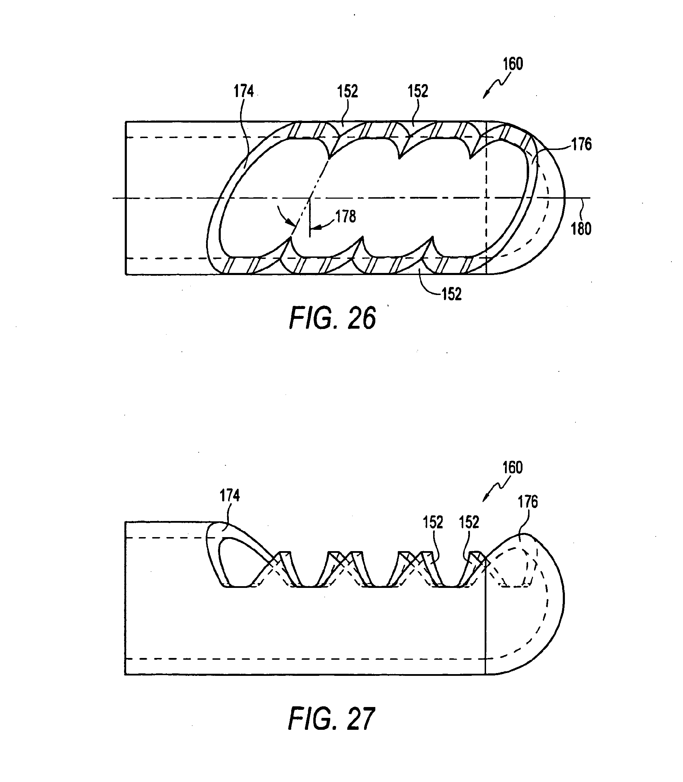

Figure 26 is a plan view of the shaver of Figure 24.

Figure 27 is a side elevational view of the shaver of Figure 24.

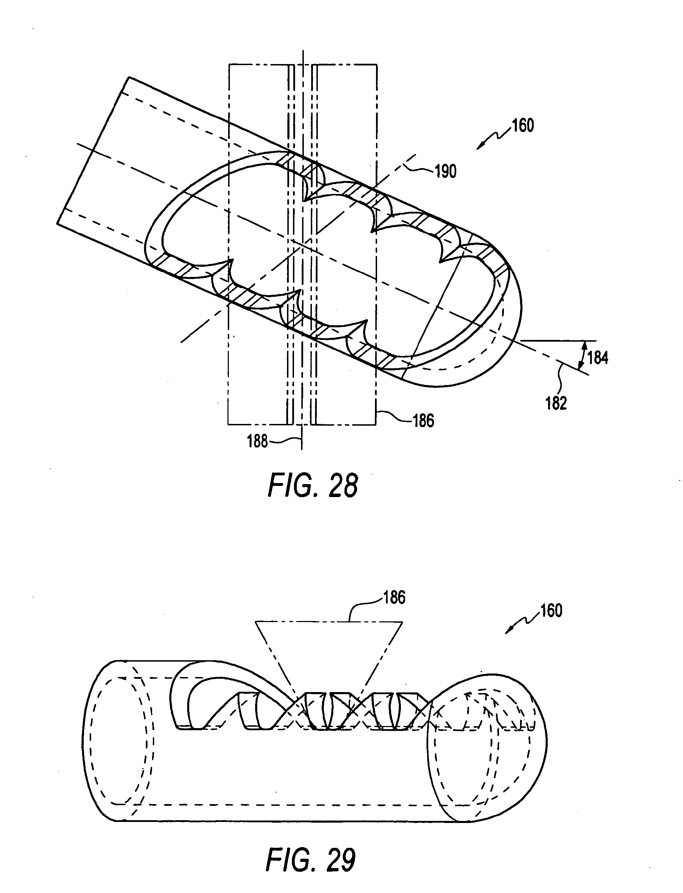

Figure 28 is a plan view of the shaver of Figure 24 reoriented in preparation for a subsequent series of grinding passes.

Figure 29 is a side elevational view of the shaver of Figure 28.

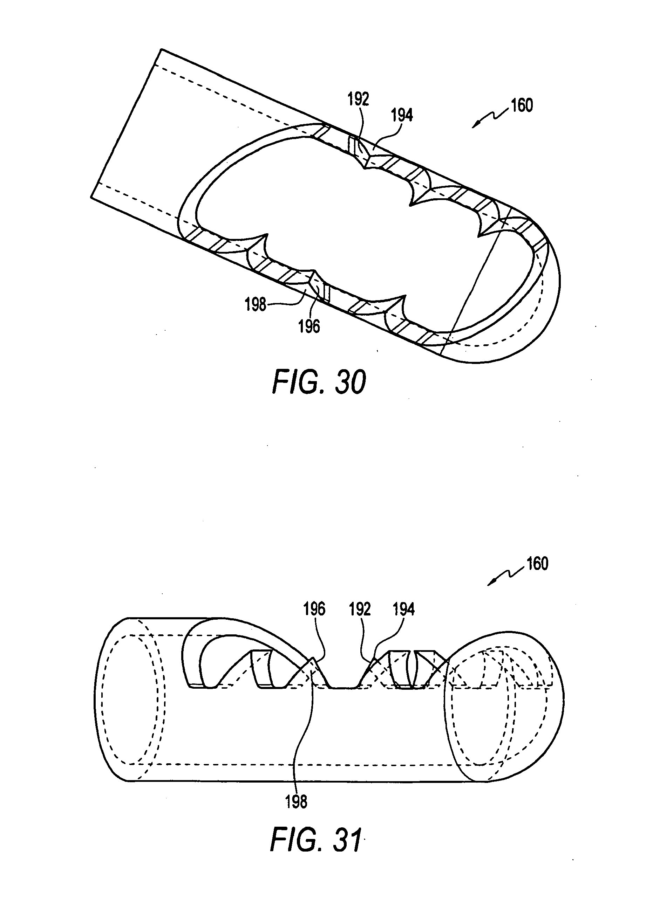

Figure 30 is a plan view of the shaver of Figure 28 after completion of a grinding pass.

Figure 31 is a side elevational view of the shaver of Figure 30.

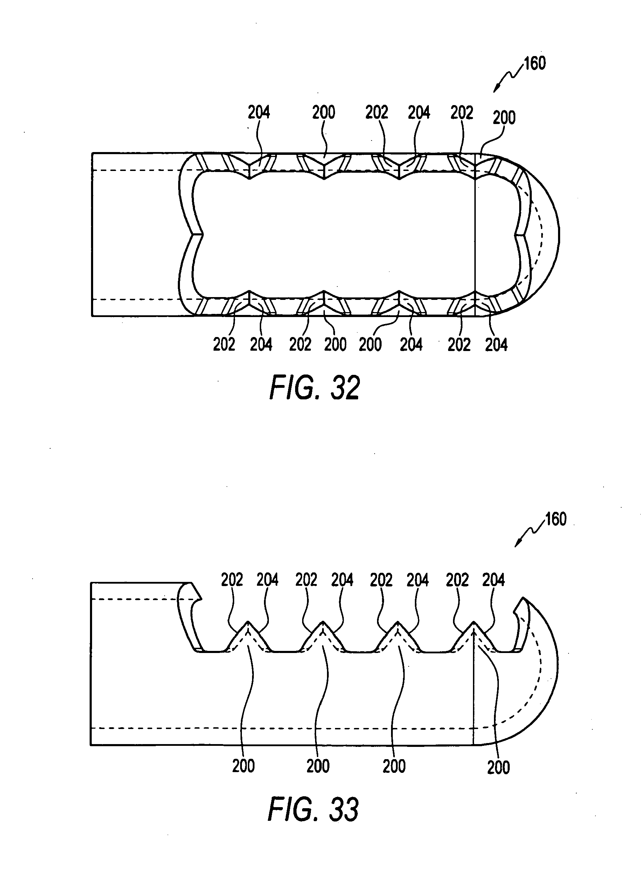

Figure 32 is a plan view of the distal portion of the tube distal end of Figure 30 after completion of a second series of grinding passes.

Figure 33 is a side elevational view of the shaver of Figure 32.

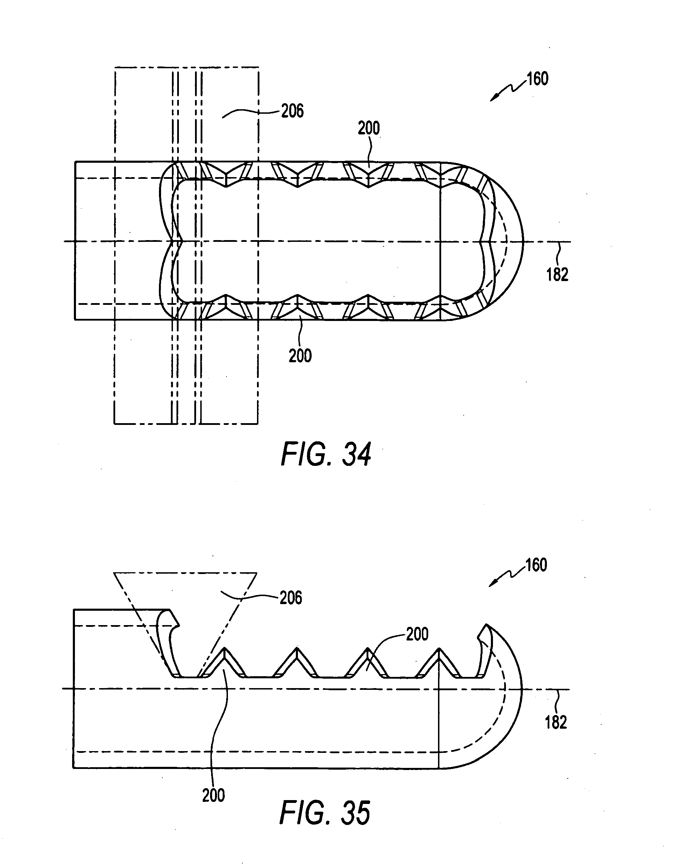

Figure 34 is a plan view of the shaver of Figure 32 prepared for a third grinding operation.

Figure 35 is a side elevational view of the shaver of Figure 34.

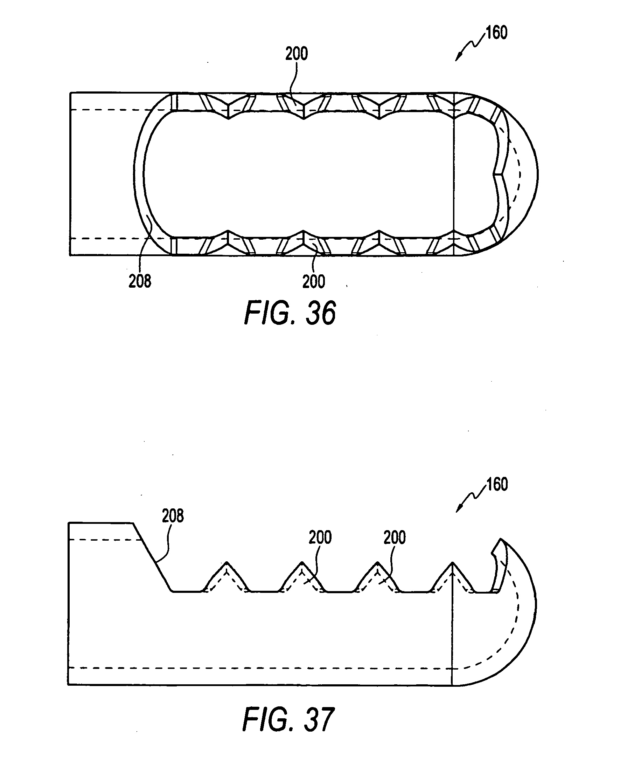

Figure 36 is a plan view of the shaver of Figure 32 after completion of a third grinding operation.

Figure 37 is a side elevational view of the shaver of Figure 36.

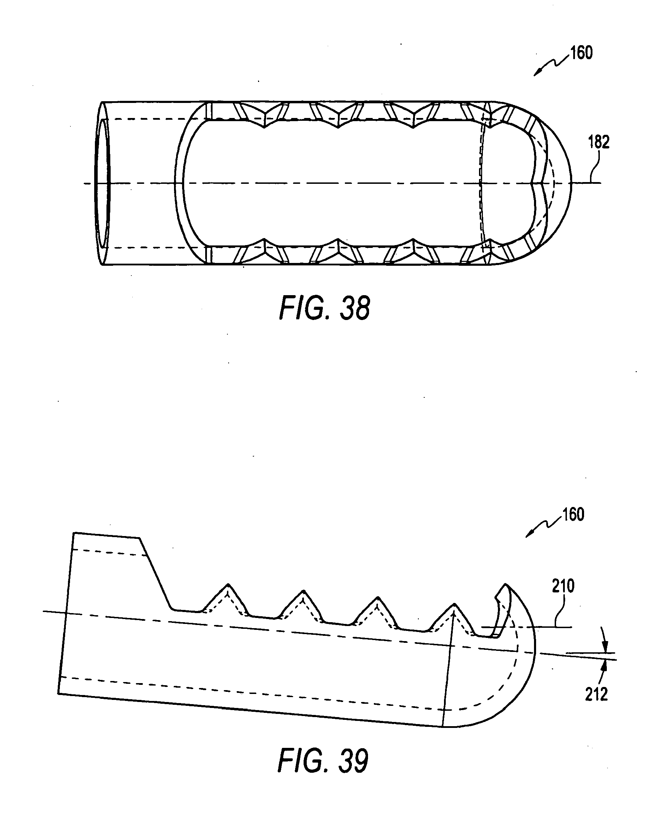

Figure 38 is a plan view of the shaver of Figure 36 oriented for a final grinding operation.

Figure 39 is a side elevational view of the shaver of Figure 38.

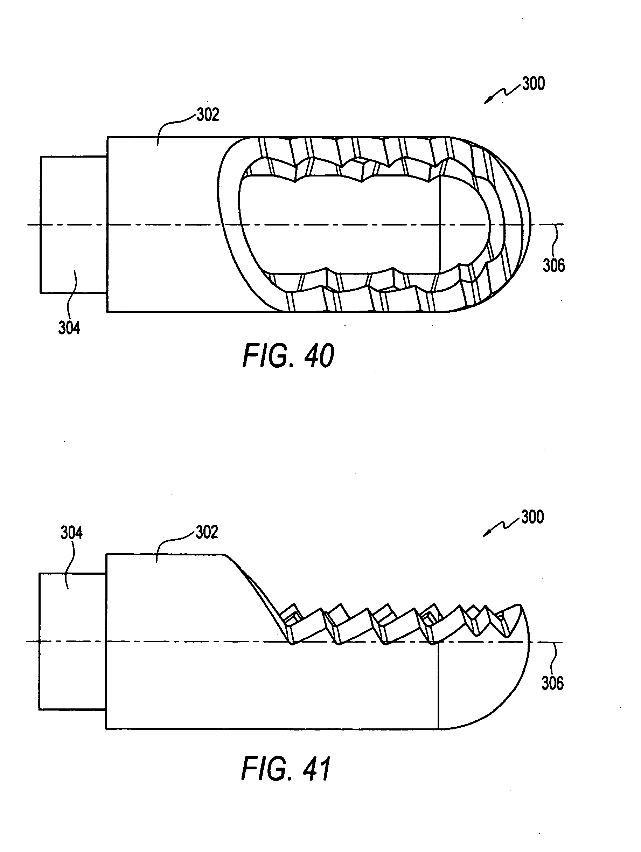

Figure 40 is a plan view of the distal end of shaver formed in accordance with another embodiment of the present invention.

Figure 41 is a side elevational view of the structures of Figure 40.

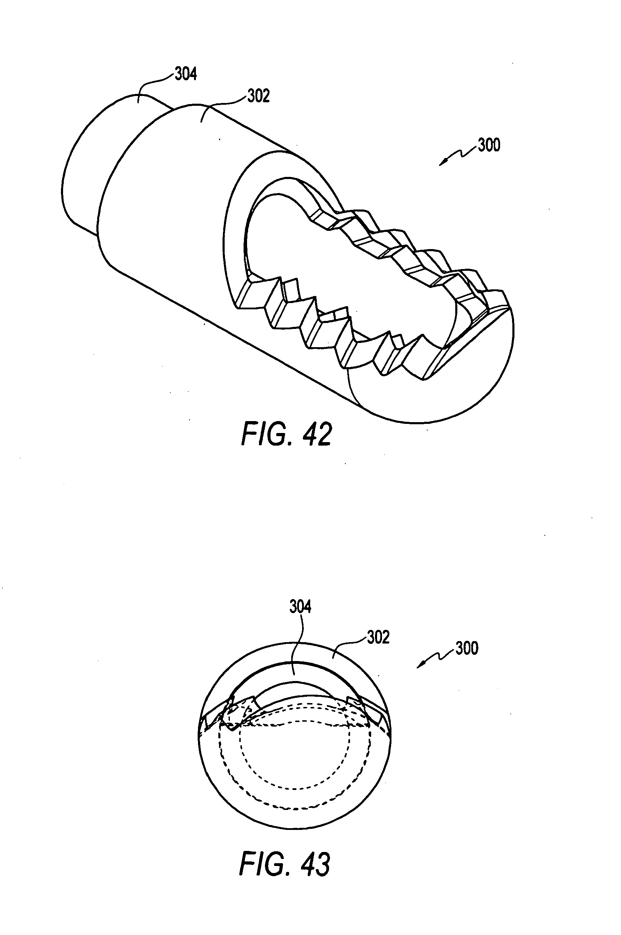

Figure 42 is a perspective view of the structures of Figure 40.

Figure 43 is a distal end view of the structures of Figure 40.

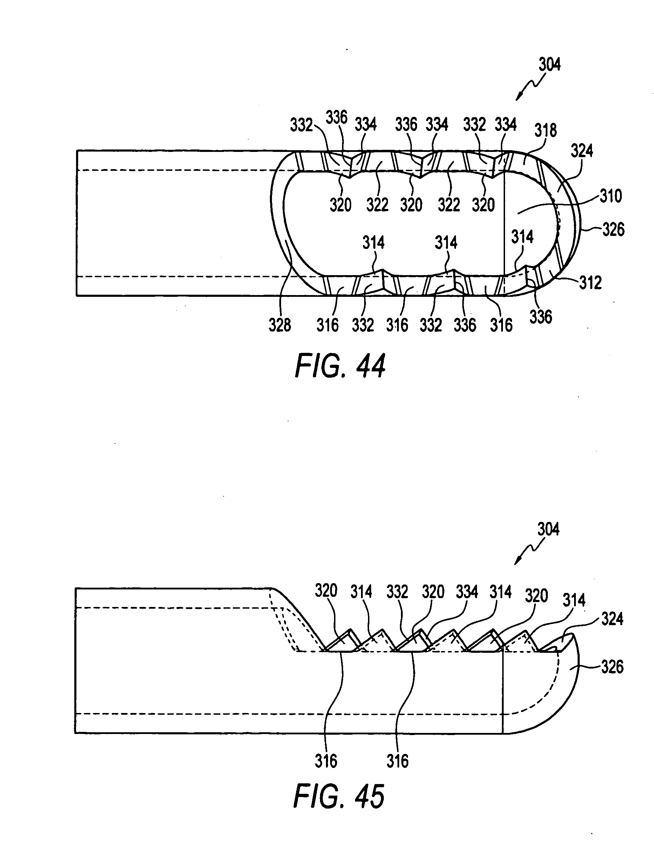

Figure 44 is a plan view of the distal end of the inner tube of the embodiment of Figure 40.

Figure 45 is a side elevational view of the structures of Figure 44.

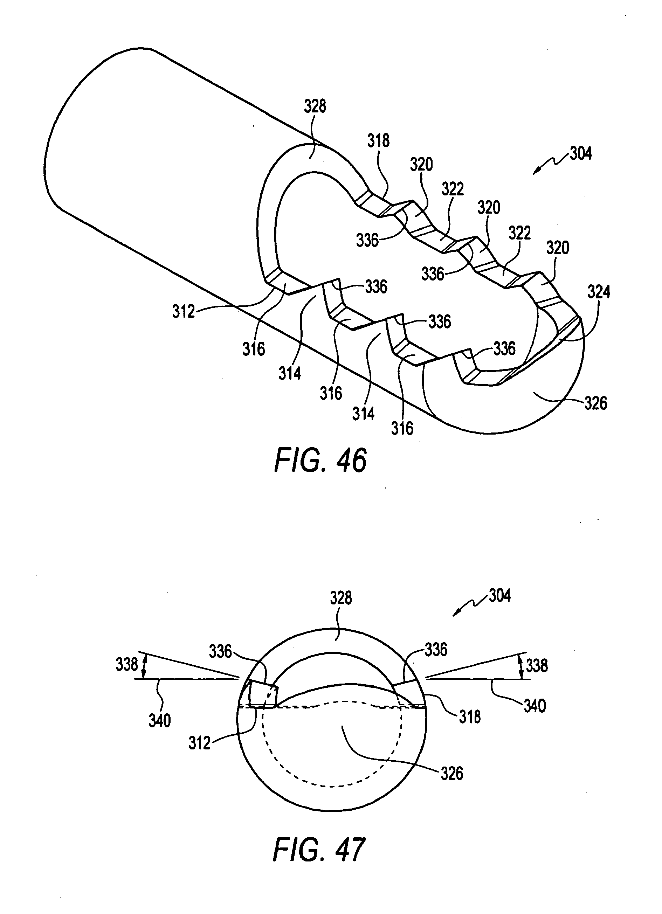

Figure 46 is a perspective view of the structures of Figure 44.

Figure 47 is a distal end view of the structures of Figure 44.

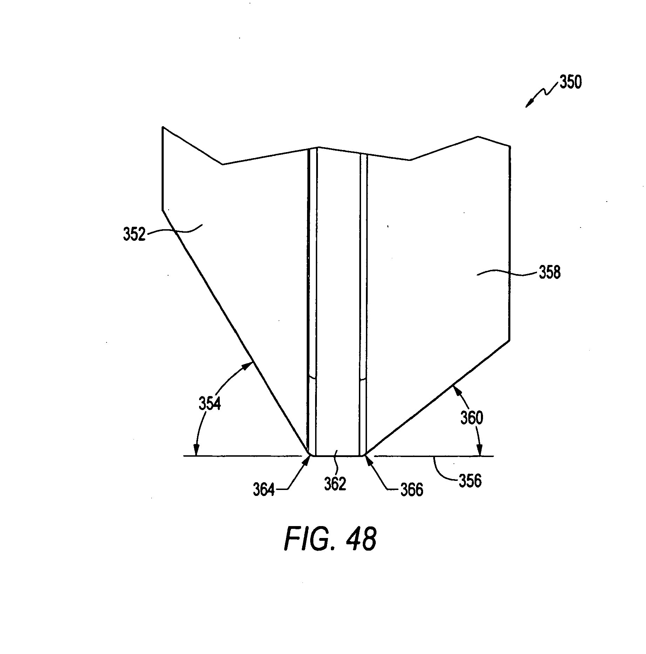

Figure 48 is an expanded tangential view of the periphery of a grinding wheel used to form the shaver of Figures 44 through 47.

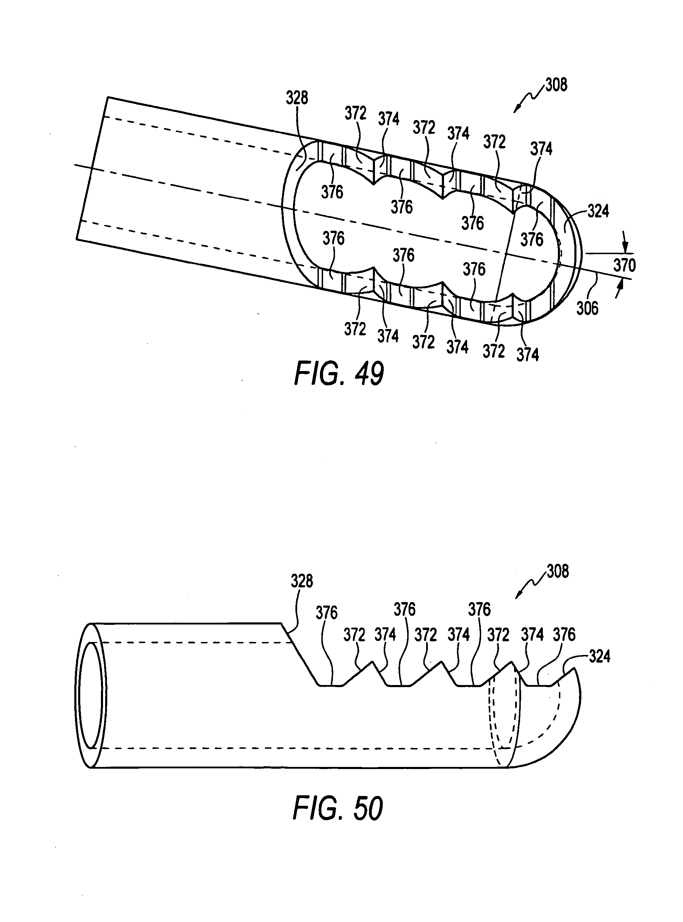

Figure 49 is a plan view of the structures of Figure 44 after completion of a first grinding operation with the cutting window partially formed, and with the tube rotated to the position used for the first grinding operation.

Figure 50 is a side elevational view of the structure of Figure 49.

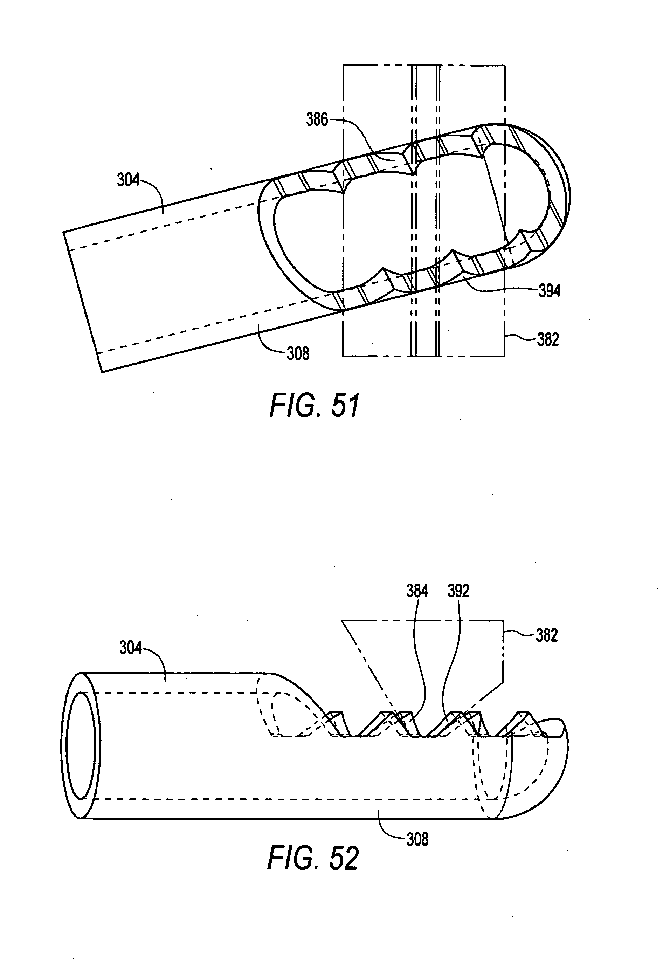

Figure 51 is a plan view of the structure of Figure 49 rotated in preparation for a second grinding operation.

Figure 52 is a side elevational view of the structure of Figure 51 with the grinding wheel periphery superimposed to show the positional relationship between the wheel and the tube.

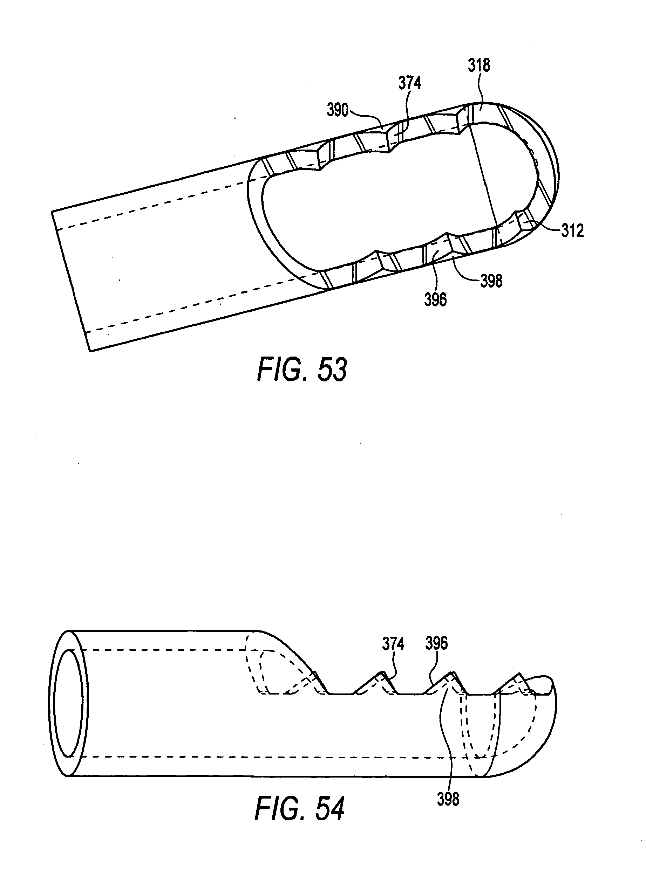

Figure 53 is a plan view of the structure of Figure 51 after completion of a second grinding operation.

Figure 54 is a side elevational view of the structure of Figure 53.

### DETAILED DESCRIPTION OF THE PREFERRED EMBODIMENTS

Figures 14 and 15 show the distal end of a shaver 79 formed in accordance with the principles of the present invention and having an inner tube 80 and an outer tube 81.

The distal end of an improved shaver inner tube with cutting edges formed in accordance with the principles of the present invention is shown in Figures 16 through 19. Shaver inner tube 80 has an inner lumen 82, and a cutting window 83 forming a first lateral cutting edge 84 having a plurality of teeth 86 spaced apart by distance 88. Shaver inner tube 80 also has a second lateral cutting edge 90 having a plurality of teeth 92 spaced apart by distance 94, distances 88 and 94 being equal. Teeth 86 are separated by flats 96 and teeth 92 are separated by flats 98. Teeth 86 and teeth 92 do not have a constant cross-section (Figure 17), but rather decrease in width as they approach the inner lumen of the tube when viewed in a plan view (Figure 16). Similarly, crests 100 of teeth 86 and crests 102 of teeth 92 are not parallel when viewed from the distal end as in Figure 18, but rather form angle 104. Teeth 86 and 92 form points 106 and 108 respectively, points 106 and 108 lying on outer surface 110 of tube 80. Distal end 112 is formed to height 114 above axis 116. Cutting window 83 is symmetrical about axis 116 when viewed in plan view as in Figure 16.

Manufacturing of the shaver inner tube according to the principles of the present invention is accomplished by a series of grinding operations using a grinding wheel having a shaped periphery. The shaver tube is angularly oriented and positioned relative to the grinding wheel and, while the grinding wheel is rotating, through relative motion between the grinding wheel and the tube, a portion of the tube is removed by the wheel. The tube is then repositioned and/or reoriented relative to the wheel, and an additional portion of the tube is removed by relative motion between the grinding wheel and the tube. A programmed sequence of such grinding operations consisting of positioning/orienting moves and grinding moves (commonly referred to as "passes") is performed to produce a finished shaver inner cutting window.

The relative position and orientation of grinding wheel 120 and of inner tube 122 during a grinding operation to form a window in accordance with the principles of the invention are shown in Figures 20 through 22. Axis 124 of wheel 120 is angularly offset from axis 126 of tube 122 of an angle 128 when viewed in a plan view (Figure 18). Axis 124 of wheel 120 is offset distance 130 from axis 126 of tube 122 when viewed in a side elevational view (Figure 21). Wheel 120 is rotated in direction 132 about axis 124. Tube 122 is moved linearly in direction 134 from a first location relative to wheel 120 to a second position, to form a shaped groove in tube 122. The shape of the groove produced is determined by the shape of the periphery of wheel 120.

Reference is now made to Figure 23, which shows an expanded tangential view of the periphery of wheel 120. The periphery has a cylindrical portion 136, a first conical portion 138 inclined at angle 140 from cylindrical portion 136, and a second conical portion 142 inclined angle 144 from cylindrical portion 136. Radius 146 is provided at the juncture between first conical portion 138 and cylindrical portion 136, and radius 148 is provided at the juncture between second conical portion 142 and cylindrical portion 136.

Figures 24 through 27 depict an inner tube with the cutting window partially formed according to the principles of the present invention. Tube 150 has a plurality of protrusions 152 formed by parallel sequential grinding passes, the grinding wheel being axially (wheel axis 124, Figure 20) repositioned between passes by distance 150 (the distance between protrusions 152). For example, surfaces 154 and 156 of protrusions 158 and 160 are formed by first conical portion 138 of wheel 120 (Figure 21), and surfaces 162 and 164 of protrusions 158 and 160 are formed by second conical portion 142 of wheel 120.

Surfaces 166, 168, 170 and 172 are formed by cylindrical portion 136 of wheel 120. Radii 146 and 148 of wheel 120 form radii between adjacent surfaces produced by a grinding pass. Surface 174 is formed by first conical portion 140 of wheel 120 (Figure 23), while surface 176 is formed by second conical portion 142 of wheel 120. Features produced by the grinding passes are angularly displaced at angle 178 with respect to axis 180 of tube 150, angle 178 being equal to angle 128 of Figure 20.

In Figures 28 and 29, tube 160 has been reoriented in preparation for the next sequence of grinding passes. Tube 160 is rotated in the plan view so that axis 182 of tube 160 is angularly displaced at angle 184 from the grinding wheel axis, angle 184 being equal to angle 178 of Figure 24. A subsequent grinding pass 186 is depicted. Centerline 188 of pass 186 intersects axis 182 at the same location as centerline 190 of a grinding pass used to form protrusions 152 (Figure 24) in the previous orientation shown in Figures 24 through 27.

Figures 30 and 31 show tube 160 after completion of grinding pass 186 shown in Figures 28 and 29. Surface 192 of tooth 194 is formed by second conical portion 142 of wheel 120 (Figure 23). Surface 196 of tooth 198 is formed by first conical portion 138 of wheel 120.

In Figures 32 and 33, the series of grinding passes parallel to pass 186 is completed. Teeth 200 of tube 160 have proximal faces 202 formed by second conical portion 142 of wheel 120, and distal faces 204 formed by first conical portion 138 of wheel 120.

In Figures 34 and 35, tube 160 has been oriented so that axis 182 of tube 160 is parallel to the grinding wheel axis. Grinding pass 206 is perpendicular to axis 182. As seen in Figures 36 and 37, first conical portion 138 of wheel 120 (Figure 23) forms surface 208 of tube 160.

In Figures 38 and 39, tube 160 is oriented for the final grinding operation, i.e., the removal of material above line 210. Tube axis 182 is parallel to the grinding wheel axis when viewed in plan view (Figure 38), but offset angle 212 when viewed in a side elevational view (Figure 39). Referring again to Figure 16, surface 112 is formed by cylindrical portion 136 of wheel 120 (Figure 23) using multiple, overlapping passes, or by another wheel having a cylindrical periphery.

Further improvement in resection efficiency is, however, possible by modification of the configurations of the cutting edge. When an inner cutting edge with teeth intersects tissue it removes tissue preferrentially in the vicinity of the teeth. Even if the inner member is operated in oscillate mode, because the teeth are symmetrically aligned about the centerline of the window, the regions of preferential tissue removal are also aligned. The amount of tissue which a tooth is able to entrap between the cutting edges is reduced since the tooth is attempting to entrap tissue in a region in which tissue was preferentially removed by the laterally opposed tooth in its previous closure of the oscillation cycle. This is particularly true in the resection of tough tissues such as meniscus or spinal disc, where the resection efficiency is heavily dependent on the ability of teeth to grab and retain tissue. By axially offsetting the teeth of one inner lateral cutting edge from those of the opposite lateral edge, the portion of tissue which is presented to a tooth when rotation is reversed in an oscillate cycle will not be in the region from which tissue was removed a tooth on the opposite side of the window. Resection efficiency can thereby be increased because the teeth are able to more effective penetrate and retain tissue in the cutting window.

According to another exemplary embodiment, the teeth formed in accordance with the present invention are asymmetrically placed about the tube axis when seen in a plan view. Referring to Figures 40 through 43, shaver 300 constructed in accordance with the principles of the present invention has an outer tube 302 and an inner tube 304, each having a cutting window with cutting edges which are asymmetrical about axis 306 of tubes 302 and 304 when viewed in a plan view (Figure 40).

Referring to Figures 44 through 47, distal end 308 of tube 304 has a cutting window 310 with a first lateral cutting edge 312 having a plurality of cutting teeth 314 separated by flat-bottomed valleys 316, and second lateral cutting edge 318 with a plurality of cutting teeth 320 separated by flat bottomed valleys 322. Inclined surface 324 intersects distal end spherical outer surface 326. Inclined surface 328 forms the proximal end of cutting window 310. Proximal surfaces 332 and distal surfaces 334 are inclined so that teeth 314 and 320 decrease in cross-sectional area with decreasing distance from tube axis 330. Surfaces 332 and 334 intersect to form tooth crests 336. As shown in Figure 47, when viewed in an axial direction, crests 336 on first cutting edge 312 and second cutting edge 318 form acute angle 338 with horizontal line 340.

Reference is now made to Figure 48, which shows the profile of the peripheral edge 350 of a grinding wheel used to form cutting edges 312 and 318 of window 310 of tube 304 (Figures 44 to 47). Edge 350 has a first conical portion 352 forming angle 354 with line 356 parallel to the axis of the grinding wheel, a second conical portion 358 forming angle 360 with line 356, and a cylindrical portion 362. Radii 364 and 366 form the junctures of first conical portion 352 and second conical portion 358 respectively with cylindrical portion 362.

Figures 49 and 50 illustrate distal end 308 of tube 304 after completion of the first sequence of grinding operations. As shown in Figure 49, tube axis 306 is angularly offset at angle 370 from the grinding wheel axis during the first grinding operation. Proximal facing angled surfaces 372 are formed by second conical portion 358 of peripheral edge 350 of the grinding wheel (Figure 48), and distal facing surfaces 374 are formed by first conical portion 352 of wheel peripheral edge 350. Surfaces 376 are formed by cylindrical portion 362 of edge 350. Surface 324 is formed by second conical portion 358 of edge 350 and surface 328 is formed by first conical portion 352 of edge 350.

Figures 51 and 52 show distal end 308 of tube 304 positioned for the second grinding operation. That is, axis 306 of tube 304 is angularly offset an angle 380 from the axis of the grinding wheel. Grinding pass 382 is positioned so that the surface produced by cylindrical portion 362 of edge 350 is coplanar with surfaces 376 formed by the first grinding operation (Figures 49 and 50). In Figure 52, the portion 384 of protrusion 386 lying within the profile of pass 382 is removed by first conical portion 352 of edge 350 (Figure 48) to form distal facing surface 374 of tooth 390 of second lateral cutting edge 318 (Figure 53). Second conical portion 358 of edge 350 (Figure 48) removes portion 392 of protrusion 394 lying within pass profile 382 to form proximal facing surface 396 of tooth 398 of first lateral cutting edge 312. Additional passes, parallel to pass 382 but axially displaced therefrom, form proximal facing surfaces 332 of teeth 314 of first lateral cutting edge 312 (Figure 44), and distal facing surfaces 334 of teeth 320 of second lateral cutting edge 318.

The characteristics of the geometry of the periphery of the grinding wheel used to form the inner cutting window strongly affect the geometry of the resulting cutting edges. For example, the wheel periphery must have a cylindrical portion to form a planar area between teeth. This allows multiple grinding passes between teeth to be made at a different range of angular orientations to the tube axis without leaving protrusions and artifacts between the teeth. The width of the cylindrical portion, and the angles of the conical portions of the grinding wheel periphery determine the range of spacing between teeth, and the geometry of the finished teeth. In the case of the asymmetric embodiment, proper selection of the angles of the conical portions allows the window to be finished complete in two multi-pass grinding operations. It is not necessary to reorient the tube so that the axis of the grinding wheel and that of the tube are parallel in order to finish the proximal and distal end of the window.

The invention herein disclosed produces an arthroscopic shaver blade with high resection efficiency due to advanced inner cutting edge tooth geometry. The shaver inner window is produced complete on a multi-axis grinding machine, the resulting cutting edges having smooth surface finishes and small edge radii.

The arthroscopic shaver formed by the method of the present invention may be employed in various surgical medical procedures such as conventional open surgeries or in other, less invasive, techniques that use cannulas or various port access devices. The shaver has applications in surgical procedures where the target tissue is ablated or shaped, and may be employed in cutting various body parts such as the knee, shoulder, hip, ankle, elbow, hand or foot. For example, the arthroscopic shaver formed according to the method of the present invention may be employed in arthroscopic surgery of a knee joint structure.

The above description and drawings illustrate preferred embodiments which achieve the objects, features and advantages of the present invention. It is not intended that the present invention be limited to the illustrated embodiments.

## Claims

1. A method of manufacturing a cutting instrument, comprising the steps of:
providing a tubular member (80, 122) having an axis (126), a proximal end and a distal end (112), and a distal opening (83) at the distal end adapted to receive anatomical tissue therethrough;
positioning the distal opening (83) of the tubular member (122) in the proximity of a grinding machine having a wheel with a periphery portion that includes a cylindrical region (136) and two conical regions (138, 142) located on opposite sides of the cylindrical region (136);
orienting the distal opening (83) at a first angle relative to the periphery of the grinding wheel (120) prior to conducting a first grinding process;
conducting a first grinding process employing the grinding wheel (120) to form a first lateral cutting edge (84) having a first plurality of teeth (86); and
reorienting the distal opening (83) at a second angle relative to the periphery of the grinding wheel to form a second lateral cutting edge (90) having a second plurality of teeth (92), wherein the steps of conducting the first and second grinding processes are conducted by employing the same grinding wheel (120);
wherein the first and second plurality of teeth (86, 92) are formed by parallel sequential grinding passes, with the grinding wheel (120) being axially repositioned between the passes.

2. The method of claim 1, wherein the two conical regions (138, 142) and the cylindrical region (136) shape the geometry of the first and second plurality of teeth (86, 92).

3. The method of claim 1, wherein the first and second plurality of teeth (86, 92) are symmetrically located relative to a longitudinal axis of the tubular member (80, 122) when viewed in a section normal to the longitudinal axis.

4. The method of claim 1, wherein the first and second plurality of teeth (86, 92) are asymmetrically located relative to a longitudinal axis of the tubular member (80, 122) when viewed in a section normal to the longitudinal axis.

5. The method of claim 1, wherein the first and second plurality of teeth (86, 92) have a pyramidal geometry.

6. The method of claim 1, wherein the cutting instrument is an inner member of a shaver blade (79) for cutting anatomical tissue.

7. The method of claim 1, wherein the two conical regions (138, 142) of the grinding wheel (120) are located at a same angle relative to a longitudinal axis of the grinding wheel (120).

8. The method of claim 1, wherein the two conical regions (138, 142) of the grinding wheel (120) are located at a different angle relative to a longitudinal axis (124) of the grinding wheel (120).

## Patentansprüche

1. Verfahren zum Herstellen eines Schneidinstruments, umfassend die folgenden Schritte:
das Bereitstellen eines röhrenförmigen Elements (80, 122), das eine Achse (126), ein proximales Ende und ein distales Ende (112) sowie eine distale Öffnung (83) am distalen Ende, die zur Aufnahme von anatomischem Gewebe angepasst ist, aufweist;
das Positionieren der distalen Öffnung (83) des röhrenförmigen Elements (122) in der Nähe einer Schleifmaschine, die ein Rad mit einem Umfangsabschnitt aufweist, der einen zylindrischen Bereich (136) und zwei an gegenüberliegenden Seiten des zylindrischen Bereichs (136) gelegene konische Bereiche (138, 142) umfasst;
das Ausrichten der distalen Öffnung (83) in einem ersten Winkel relativ zum Umfang des Schleifrads (120) vor der Durchführung eines ersten Schleifvorgangs;
das Durchführen eines ersten Schleifvorgangs unter Verwendung des Schleifrads (120), um eine erste seitliche Schneidkante (84) mit einer ersten Mehrzahl von Zähnen (86) zu bilden; und
das erneute Ausrichten der distalen Öffnung (83) in einem zweiten Winkel relativ zum Umfang des Schleifrads, um eine zweite seitliche Schneidkante (90) mit einer zweiten Mehrzahl von Zähnen (92) zu bilden, wobei die Schritte des Durchführens des ersten und des zweiten Schleifvorgangs unter Verwendung desselben Schleifrads (120) ausgeführt werden;
wobei die erste und die zweite Mehrzahl von Zähnen (86, 92) durch parallele sequentielle Schleifdurchgänge gebildet werden, wobei das Schleifrad (120) zwischen den Durchgängen axial repositioniert wird.

2. Verfahren nach Anspruch 1, wobei die zwei konischen Bereiche (138, 142) und der zylindrische Bereich (136) die Geometrie der ersten und der zweiten Mehrzahl von Zähnen (86, 92) formen.

3. Verfahren nach Anspruch 1, wobei die erste und die zweite Mehrzahl von Zähnen (86, 92) symmetrisch in Bezug auf eine Längsachse des röhrenförmigen Elements (80, 122) gelegen sind, wenn sie in einem Schnitt normal zur Längsachse betrachtet werden.

4. Verfahren nach Anspruch 1, wobei die erste und die zweite Mehrzahl von Zähnen (86, 92) asymmetrisch in Bezug auf eine Längsachse des röhrenförmigen Elements (80, 122) gelegen sind, wenn sie in einem Schnitt normal zur Längsachse betrachtet werden.

5. Verfahren nach Anspruch 1, wobei die erste und die zweite Mehrzahl von Zähnen (86, 92) eine pyramidenförmige Geometrie aufweisen.

6. Verfahren nach Anspruch 1, wobei das Schneidinstrument ein inneres Element einer Schabklinge (79) zum Schneiden von anatomischem Gewebe ist.

7. Verfahren nach Anspruch 1, wobei die zwei konischen Bereiche (138, 142) des Schleifrads (120) in einem gleichen Winkel in Bezug auf eine Längsachse des Schleifrads (120) gelegen sind.

8. Verfahren nach Anspruch 1, wobei die zwei konischen Bereiche (138, 142) des Schleifrads (120) in einem unterschiedlichen Winkel in Bezug auf eine Längsachse (124) des Schleifrads (120) gelegen sind.

## Revendications

1. Procédé de fabrication d'un instrument de coupe, comprenant les étapes consistant à :
fournir un organe tubulaire (80, 122) présentant un axe (126), une extrémité proximale et une extrémité distale (112), et une ouverture distale (83) à l'extrémité distale, adaptée pour recevoir à travers elle du tissu anatomique ;
positionner l'ouverture distale (83) de l'organe tubulaire (122) à proximité d'une machine de meulage-rectification ayant une roue munie d'une partie périphérique incluant une région cylindrique (136) et deux régions coniques (138, 142), situées sur des côtés opposés de la région cylindrique (136) ;
orienter l'ouverture distale (83) selon un premier angle, par rapport à la périphérie de la roue de meulage-rectification (120), avant de procéder à un premier processus de meulage-rectification ;
effectuer un premier processus de meulage-rectification en utilisant la roue de meulage-rectification (120), pour former un premier bord de coupe latéral (84) comprenant une première pluralité de dents (86) ; et
réorienter l'ouverture distale (83) selon un deuxième angle, par rapport à la périphérie de la roue de meulage-rectification, pour former un deuxième bord de coupe latéral (90) ayant une deuxième pluralité de dents (92), dans lequel les étapes d'exécution des premier et deuxième processus de meulage-rectification sont effectuées en utilisant la même roue de meulage-rectification (120) ;
dans lequel les premières et deuxième pluralité de dents (86, 92) sont formées par des passes de meulage-rectification séquentielles parallèles, la roue de meulage-rectification (120) étant repositionnée axialement entre les passes.

2. Procédé selon la revendication 1, dans lequel les deux régions coniques (138, 142) et la région cylindrique (136) façonnent la géométrie des première et deuxième pluralité de dents (86, 92).

3. Procédé selon la revendication 1, dans lequel les première et deuxième pluralités de dents (86, 92) sont situées symétriquement par rapport à un axe longitudinal de l'organe tubulaire (80, 122), lorsqu'on observe en vue en coupe perpendiculaire à l'axe longitudinal.

4. Procédé selon la revendication 1, dans lequel les première et deuxième pluralités de dents (86, 92) sont situées asymétriquement par rapport à un axe longitudinal de l'organe tubulaire (80, 122), lorsqu'on observe en vue en coupe perpendiculaire à l'axe longitudinal.

5. Procédé selon la revendication 1, dans lequel les première et deuxième pluralités de dents (86, 92) ont une forme géométrique pyramidale.

6. Procédé selon la revendication 1, dans lequel l'instrument de coupe est un organe intérieur d'une lame d'organe de résection (79) pour effectuer une résection d'un tissu anatomique.

7. Procédé selon la revendication 1, dans lequel les deux régions coniques (138, 142) de la roue de meulage-rectification (120) sont situées selon un même angle par rapport à un axe longitudinal de la roue de meulage-rectification (120).

8. Procédé selon la revendication 1, dans lequel les deux régions coniques (138, 142) de la roue de meulage-rectification (120) sont situées selon un angle différent par rapport à un axe longitudinal (124) de la roue de meulage-rectification (120).
